Europäisches Patentamt

European Patent Office

Office européen des brevets

(11) Publication number: **0 432 882 A2**

# EUROPEAN PATENT APPLICATION

(21) Application number: 90311527.7

(51) Int. Cl.⁵: **A61F 13/15**, A61F 13/46

(22) Date of filing: 19.10.90

(30) Priority: 19.10.89 GB 8923575

(43) Date of publication of application:
19.06.91 Bulletin 91/25

(84) Designated Contracting States:
DE ES FR IT

(71) Applicant: DISPOSABLES AND DIAPER
DEVELOPMENTS LIMITED
Independence House, Vale Road, Otterspool
Stockport SK6 3LE(GB)

(72) Inventor: Shipley, Arthur Roger
8 Oakhill Drive, Prestatyn
Clwyd,Wales(GB)

(74) Representative: Atkinson, Peter Birch
MARKS & CLERK Suite 301 Sunlight House
Quay Street
Manchester M3 3JY(GB)

(54) Sanitary articles.

(57) A sanitary article (e.g. a disposable diaper) comprises an absorbent layer sandwiched between a liquid permeable and liquid impermeable layer. A discrete area of the liquid permeable layer (which is the layer which will be positioned in use adjacent the body) is treated with a surface active agent so as to facilitate passage of body fluid through only that area of the layer. Thus, the body fluids which pass into the absorbent layer are prevented or inhibited from returning back through the untreated areas of the permeable layer.

EP 0 432 882 A2

## SANITARY ARTICLES

The present invention relates to sanitary articles which are for use in absorbing body fluids. The invention relates particularly, although by no means exclusively, to disposable nappies (diapers).

It is well known that disposable nappies comprise absorbent material (e.g. defibrated wood pulp) sandwiched between a porous layer (e.g a non-woven polypropylene or polyester) and a water impermeable layer (e.g. polyethylene). Disposable nappies have traditionally been constructed to give satisfactory performance in total absorbency terms, when used by both sexes. However, these traditional nappy constructions have not specifically identified the anatomic difference between boys and girls anc therefore do not have the inherent ability to accept the initial flow or urine in the anatomically gender specific target areas of the nappy. Furthermore, the permeable layer of the nappy has an initial resistance to the passage of body fluid through the web fibres.

It is known that non woven polypropylene and polyester materials generically are treated with surface active agents in low concentrations at the time of manufacture to achieve a degree of permeability. This process has traditionally lacked consistency and has produced significant variability in permeability performance. We have also found that there is the additional disadvantage in that, because the full area of the porous layer in the sanitary article is treated with the surface active agent, body fluids which has through the porous layer are able to pass back through this layer over any portion of the area thereof.

It is therefore an object of the present invention to obviate or mitigate the above mentioned disadvantages.

According to the present invention there is provided a sanitary article comprising a liquid absorbent layer provided between an external liquid impermeable layer and an internal liquid permeable layer wherein at least one discrete area of the liquid permeable layer is treated with a non-irritant surface active agent which has the effect of facilitating the passage of body fluids through the permeable layer into the absorbent layer of the article.

The treatment of the permeable layer with the surface active agent is designed to have the effect of changing the surface tension of the body fluid when this fluid is in contact with the permeable layer of the article) so that this fluid may pass more readily through to the absorbent layer only through the treated area thereof.

Thus, any body fluid which enters the absorbent material and "travels" therealong is prevented or at least inhibited from passing back through the porous layer over any portion of the area thereof which is not treated with the surface active agent. Thus, there is only minimal return of body fluids back through the porous layer.

In the case where the sanitary article is a disposable nappy, it is possible for the surface active agent to be targeted into the anatomically correct zones of the nappy to give a preferential area of absorbency to suit the anatomy of a boy or a girl. Thus, for example, it is possible to produce nappies for use by girls by providing the surface active agent only over a central area of the permeable material. Alternatively, it is possible to produce nappies for use by boys, by providing the surface active agent over an area of the permeable web which corresponds to the front of the nappy when worn by the child.

Alternatively unisex nappies can be produced by applying the surface active agent continuously to the full length of the absorbent area of the nappy.

The treatment, of the permeable layer, can be applied to either one or both sides of the web and may also be intermittent or continuous as desired.

A further aesthetic feature can be the coloration of the surface active agent prior to or during the application to the permeable layer, thus clearly identifying the position of the treatment on the fully constructed nappy.

In conventional manner, the absorbent layer of the nappy may comprise defibrated wood pulp, the outer liquid impermeable layer may be polyethylene, and the inner liquid permeable layer may be a non woven material such as polypropylene or polyester. The disposable nappy may be of conventional "hour glass" configuration.

Preferably, the liquid impermeable layer, absorbent layer, and liquid permeable layer are assembled together into the form of the sanitary article before the liquid permeable layer is treated with the surface active agent. The treatment with the surface active agent may be effected in a number of ways. One method is to use a non-contact pressure spray coating method. Alternatively, a contact method such as flexographic printing or rotary drum transfer coating method may be used. Alternatively, it is possible to treat the liquid permeable layer with the surface active agent prior to assembly into the sanitary article.

The liquid permeable layer may, for example, be a non-woven polypropylene or polyester material. It is possible for the liquid permeable layer to be a generally hydrophobic membrane which becomes relatively hydrophilic in the area treated with the surface active agent.

Surface active agents which may be used include
Polyoxyethylene (20) Sorbitan Mono Oleate
Octylphenoxypolyethoxyethanol Nonionic Surfactant
Nonylphenoxypoly (Ethylenoxy) Ethanol

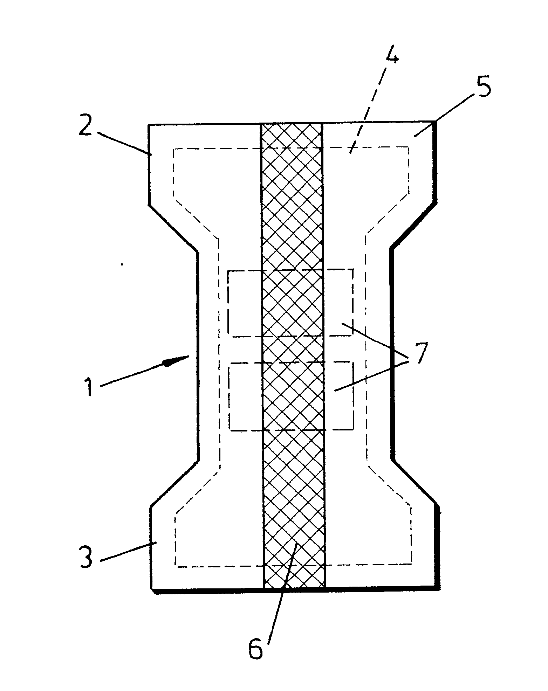

The invention will be further described by way of example only with reference to the accompanying drawing which illustrates one embodiment of disposable diaper in accordance with the invention.

The illustrated diaper 1 is of generally hourglass configuration and includes enlarged end portions 2 and 3 which (when the diaper is worn) provide waist bands. The diaper is of generally conventional construction and comprises an outer liquid impermeable layer (not shown) an intermediate absorbent layer 4, and a liquid permeable layer 5. This layer 5 is treated over a generally rectangular area 6 with a surface active agent. This area 6 extends generally centrally along the diaper between opposite ends thereof at least over the length of the absorbent pad 4. Thus, when the diaper is worn by either a male or female child, a portion of the area 6 is adjacent the urethral orifice so that urine is directed immediately into the area 6. Urine will pass readily through the area 6 into the absorbent pad 4. However, any urine which "travels" through the pad 4 will be prevented or inhibited from returning back through the layer 5, at least in the areas thereof outside the rectangle 6.

In an alternative construction of the diaper, the surface active agent may be provided in gender specific areas such as schematically illustrated by the rectangular areas 7.

Although the present invention has been described specifically with respect to disposable nappies, the invention is also applicable to other disposable absorbent articles such as sanitary towels or surgical dressings or the like.

## Claims

1. A sanitary article comprising a liquid absorbent layer provided between an external liquid impermeable layer and an internal liquid permeable layer wherein at least one discrete area of the liquid permeable layer is treated with a non-irritant surface active agent which has the effect of facilitating the passage of body fluids through the permeable layer into the absorbent layer of the article.

2. An article as claimed in claim 1, which is a disposable diaper.

3. An article as claimed in claim 2, wherein said at least one discrete area is at least one gender specific area.

4. An article as claimed in claim 2, wherein said at least one discrete area is an elongate area extending centrally of the diaper between opposite ends thereof such that when the diaper is worn by either a male or female child a portion of the elongate area is disposed adjacent the urethral orifice.

5. An article as claimed in any one of claims 1 to 4, wherein the area of the porous layer treated with the surface active agent is coloured as compared to the untreated area of the layer.

6. An article as claimed in any one of claims 1 to 5, wherein the porous layer has been treated with the surface active agent after the liquid impermeable layer, absorbent layer and liquid permeable layer have been assembled together.

7. A sanitary article substantially as hereinbefore described with reference to the accompanying drawings.